# DEMANDE DE BREVET EUROPEEN

(11) **EP 4 659 860 A1**
(43) Date de publication de la demande: **10.12.2025**
(21) Numéro de dépôt: 25181100.6
(22) Date de dépôt: 05.06.2025
(51) Int. Cl.: B01L 3/00

(54) **DISPOSITIF ET PROCÉDÉ DE TRAITEMENT DE FLUIDE**

(30) Priorité: 07.06.2024 BE 202405336
(71) Demandeur: Elysia, 4031 Liège (BE)
(72) Inventeur: CLERDY, Julien, 4000 Liège (BE); ROYEN, François, 4357 Donceel (BE); THONON, David, 4130 Tilff (BE)
(74) Mandataire: Gevers Patents

(57) **Abrégé**

L'invention concerne un dispositif (520) de traitement de fluide comprenant : une cassette (500) comprenant une tubulure (501) préassemblée, la cassette (500) présentant une surface (530) plane pour recevoir la tubulure (501), la tubulure (501) comprenant au moins un tube souple (104) pouvant être pincé ; au moins une vanne à pincement (100) comprenant un ergot mobile (101) et une butée fixe (103) pour pincer le tube souple (104) pour y contrôler un débit de fluide ; dans lequel la vanne à pincement (100) est couplée mécaniquement avec la cassette (500) de sorte que l'ergot mobile (101) de la vanne à pincement (100) décrive une trajectoire parallèle à la surface (530) plane de la cassette lors d'une activation de la vanne à pincement (100) pour pincer le tube souple (104), et dans lequel la tubulure (501) peut être mise en place et remplacée facilement, rapidement, et à moindre coût. L'invention concerne également un procédé de traitement de fluide permettant de mettre en place et remplacer facilement, rapidement, et à moindre coût une tubulure (501) comprise dans un dispositif (520) de traitement de fluide selon l'invention.

## Description

### Domaine technique

La présente invention concerne le domaine du traitement de fluides pour l'analyse et la synthèse de composés chimiques, en particulier le domaine de l'analyse et la synthèse de composés radiopharmaceutiques, via des dispositifs et procédés de traitement fluidiques et micro-fluidiques permettant la manipulation et le contrôle précis de débits de fluides. De préférence, l'invention concerne de tels dispositifs destinés à un faible nombre d'utilisations, voire à un usage unique.

### Etat de la technique

Dans le domaine de l'analyse et la synthèse de composés radiopharmaceutiques, des schémas fluidiques complexes sont généralement réalisés grâce à un manifold. Dans le cadre du présent document, le terme « manifold » désigne un système de distribution et de contrôle d'un débit d'un ou plusieurs fluides, comme par exemple un jeu de raccordements et de vannes permettant diverses combinaisons de branchement de tubes, un fluide pouvant être un liquide ou un gaz. Un manifold peut comprendre les éléments suivants : des tubes pour transporter un fluide ; des jonctions pour raccorder différents tubes ; des vannes pour contrôler, i.e. réguler ou interrompre, un débit d'un fluide ; des connecteurs pour raccorder des tubes à des équipements. Un manifold implémente un « schéma fluidique », i.e. une configuration de tubes, jonctions, vannes, connecteurs, etc. permettant de réaliser un procédé donné de traitement d'un ou plusieurs fluides.

L'utilisation de manifolds classiques, où le fluide circulant dans le manifold est en contact avec des vannes du manifold, peut présenter plusieurs inconvénients. Tout d'abord, il peut être difficile de nettoyer ces vannes, ce qui peut mener à des risques de contamination d'un fluide par un autre fluide circulant précédemment dans le manifold. De plus, il peut y avoir des portions de fluide résiduelles dans les vannes, ce qui peut mener à des pertes de fluides, ces fluides perdus étant également désignés comme des « volumes morts ». Enfin, il peut y avoir des problèmes de stabilité chimique car les fluides circulant dans le manifold peuvent réagir avec les matériaux constituant les vannes et jonctions. Dans le cas d'un faible nombre d'utilisations, voire d'un usage unique, d'un manifold classique, où le fluide circulant dans le manifold est en contact avec des vannes du manifold, il peut être nécessaire de se débarrasser de ces vannes après leur utilisation, ce qui peut poser plusieurs problèmes, comme par exemple un grand volume de déchets générés, ou encore un coût important de remplacement du manifold. Dans le cas où le fluide circulant dans le manifold contient un composé radioactif, générer un grand volume de déchets potentiellement radioactifs peut être problématique dans la mesure où ces déchets potentiellement radioactifs doivent faire l'objet d'un traitement spécifique qui peut être coûteux.

Afin de surmonter ces inconvénients, il est possible de séparer les vannes proprement dites des tubes transportant le fluide, ce qui a mené à la création de vannes dites « à pincement de tube », où une vanne contrôle un débit d'un fluide dans un tube souple par pincement du tube, ce qui permet d'éviter tout contact direct entre la vanne proprement dite et le fluide transporté, éliminant ainsi les problèmes de contamination et également de volumes morts au niveau des vannes d'un manifold.

Il existe au moins deux grandes catégories de vannes à pincement de tube, ci-après également dénommées plus simplement « vannes à pincement ». Tout d'abord, les vannes à pincement pneumatiques, où le mécanisme de pincement de la vanne est actionné au moyen d'air comprimé. Ensuite, les électrovannes à pincement, qui comprennent un électroaimant et un ressort de rappel, et utilisent un courant électrique traversant l'électroaimant afin d'actionner le mécanisme de pincement de la vanne.

Toutefois, plusieurs problèmes demeurent avec l'utilisation de vannes à pincement afin de contrôler un débit d'un fluide dans un ou plusieurs tubes ou dans un réseau de tubes, ci-après également dénommés « tubulure ». En effet, il peut ne pas être facile de séparer la tubulure du mécanisme de pincement de la vanne, afin de mettre en place ou de remplacer la tubulure, comme par exemple dans le cas d'une tubulure pour un faible nombre d'utilisations ou à usage unique. De plus, dans le cas d'un dispositif de traitement de fluide comprenant une tubulure pour un faible nombre d'utilisations ou à usage unique, il peut être souhaitable d'avoir une tubulure préassemblée, qui peut ainsi être plus facilement et plus rapidement mise en place et remplacée. En particulier, pour certaines applications comme une synthèse de composés pharmaceutiques ou radiopharmaceutiques, il peut être nécessaire de manipuler la tubulure dans un isolateur permettant la mise en place de la tubulure à travers des gants, comme par exemple un dispositif de type « boîte à gants ». Dans ce cas, il peut être difficile de manipuler la tubulure et une tubulure qui ne serait pas préassemblée pourrait être difficile à mettre en place dans un dispositif de traitement de fluide. Enfin, il peut être souhaitable qu'un tel dispositif comprenant une tubulure pour un faible nombre d'utilisations ou à usage unique nécessite peu de ressources pour être fabriqué, afin de limiter un coût de fabrication et/ou de remplacement de la tubulure.

Le document US5901745A décrit un dispositif comprenant une série de vannes à pincement et leurs tubes souples respectifs. Un inconvénient de ce dispositif est qu'il ne permet pas une mise en place ou un remplacement facile et rapide de la tubulure. Ainsi, il est nécessaire de démonter l'ensemble du dispositif pour pouvoir mettre en place ou remplacer la tubulure, ce qui peut entraîner un coût important.

Il existe donc un besoin pour un dispositif de traitement de fluide comprenant une tubulure pouvant être mise en place et remplacée facilement, rapidement, et à moindre coût.

### Exposé de l'invention

Selon un premier aspect, un objet de la présente invention est de fournir un dispositif de traitement de fluide comprenant une tubulure pouvant être mise en place et remplacée facilement, rapidement, et à moindre coût.

À cet effet, l'invention propose un dispositif de traitement de fluide comprenant :
- une cassette comprenant une tubulure préassemblée, la cassette présentant (ou ayant) une surface essentiellement plane pour recevoir la tubulure, la tubulure comprenant au moins un tube souple pouvant être pincé (ou apte à être pincé) ;
- au moins une vanne à pincement comprenant un ergot (ou patte) mobile et une butée fixe pour pincer le tube souple pour y contrôler un débit de fluide (i.e. pour contrôler un débit de fluide dans ledit tube souple) ;
dans lequel l'au moins une vanne à pincement est couplée mécaniquement avec la cassette de sorte que l'ergot mobile de l'au moins une vanne à pincement décrive une trajectoire essentiellement parallèle à la surface essentiellement plane de la cassette lors d'une activation de l'au moins une vanne à pincement pour pincer le tube souple.

Dans le cadre du présent document, le terme « tubulure » désigne un ou plusieurs tubes, de préférence un réseau de tubes. Un tel réseau de tubes peut comprendre au moins une ramification. Dans le cadre du présent document, une « cassette » désigne un corps de support d'une tubulure. Une telle cassette est de préférence un boîtier ou une cartouche qui peut être facilement monté(e) ou démonté(e). Une telle cassette a de préférence une forme plate. Une vanne à pincement de tube, aussi appelée plus simplement « vanne à pincement » dans le présent document, permet de contrôler, i.e. réguler ou interrompre, un débit d'un fluide dans un tube souple d'une tubulure par pincement dudit tube souple.

La vanne à pincement comprise dans le dispositif selon l'invention est apte à pincer le tube souple entre l'ergot mobile et la butée fixe pour contrôler un débit de fluide circulant dans le tube souple. Autrement dit, le tube souple compris dans la tubulure est apte à être pincé par la vanne à pincement. Plus précisément, le tube souple est apte à être pincé entre l'ergot mobile et la butée fixe de la vanne à pincement.

La tubulure comprise dans le dispositif de traitement de fluide selon la présente invention peut être mise en place et remplacée facilement, rapidement, et à moindre coût. En effet, la cassette comprenant la tubulure est distincte de la vanne à pincement comprenant l'ergot mobile et la butée fixe. Ainsi, la cassette comprenant la tubulure préassemblée peut être couplée mécaniquement avec (ou montée sur) la vanne à pincement. De préférence, la cassette peut être couplée de manière amovible avec la vanne à pincement. En particulier, la cassette peut être couplée avec (ou découplée de) la vanne à pincement sans devoir démonter la vanne à pincement. Autrement dit, la cassette comprenant la tubulure préassemblée est prête à l'emploi (« plug and play »). De cette manière, la cassette peut être manipulée, i.e. mise en place et remplacée (ou simplement retirée), indépendamment de l'au moins une vanne à pincement.

De plus, lors de l'activation de la vanne à pincement, l'ergot mobile compris dans la vanne à pincement décrit une trajectoire essentiellement parallèle à la surface essentiellement plane de la cassette. De plus, dans le cas où le dispositif de traitement de fluide selon l'invention comprend plusieurs vannes à pincement, ces vannes sont situées sur un plan essentiellement parallèle à la surface essentiellement plane de la cassette. La trajectoire de l'ergot mobile de chaque vanne à pincement comprise dans le dispositif selon l'invention ne suit donc pas une direction essentiellement perpendiculaire à la surface plane de la cassette, laissant cette direction perpendiculaire libre pour coupler la tubulure comprise dans la cassette à chaque vanne à pincement comprise dans le dispositif selon l'invention.

Ainsi, la cassette comprenant la tubulure peut être mise en place en un seul mouvement suivant une trajectoire dont la direction est essentiellement perpendiculaire à la surface essentiellement plane de la cassette et orientée vers l'au moins une vanne à pincement comprise dans le dispositif selon l'invention, afin de coupler la tubulure avec ladite au moins une vanne à pincement. La tubulure comprise dans la cassette peut donc être couplée mécaniquement avec l'ensemble des vannes à pincement comprises dans le dispositif selon l'invention en une seule opération. En particulier, il n'est pas nécessaire d'ajuster individuellement la tubulure à chacune de la ou desdites vannes à pincement.

De même, la tubulure comprise dans la cassette peut être découplée mécaniquement de l'ensemble des vannes à pincement comprises dans le dispositif selon l'invention en un seul mouvement, suivant une trajectoire dont la direction est essentiellement perpendiculaire à la surface essentiellement plane de la cassette et orientée à l'opposé de l'au moins une vanne à pincement comprise dans le dispositif selon l'invention. La tubulure comprise dans la cassette peut donc être découplée mécaniquement de l'ensemble des vannes à pincement comprises dans le dispositif selon l'invention en une seule opération. En particulier, il n'est pas nécessaire de découpler individuellement la tubulure de chacune de la ou desdites vannes à pincement.

Dès lors, la tubulure comprise dans le dispositif selon l'invention peut être mise en place et remplacée facilement et rapidement. De plus, comme un coût de mise en place ou de remplacement de la tubulure peut être proportionnel au temps mis par un opérateur pour réaliser une telle opération de mise en place ou de remplacement de la tubulure, le fait de pouvoir réaliser ces opérations rapidement permet de les effectuer à moindre coût.

La tubulure est préassemblée dans la cassette. Le fait que la tubulure soit préassemblée dans la cassette signifie que la tubulure est assemblée avec la cassette avant que la cassette soit couplée avec l'au moins une vanne à pincement. La cassette ne comprend pas d'ergot mobile ni de butée fixe, ces deux derniers éléments étant compris dans une vanne à pincement distincte de la cassette. Autrement dit, la cassette comprenant la tubulure est indépendante de la vanne à pincement comprenant l'ergot mobile et la butée fixe. Le fait que la tubulure soit préassemblée dans la cassette permet une mise en place et un remplacement rapide et simple de la tubulure sur le dispositif selon l'invention.

Un autre avantage d'avoir une tubulure préassemblée dans la cassette est de pouvoir contraindre ou contrôler une ou plusieurs dimensions caractéristiques de la tubulure, comme une longueur de la tubulure ou un diamètre de la tubulure.

Par ailleurs, le fait que la tubulure soit préassemblée dans la cassette permet à un opérateur de mettre en place ou de remplacer la cassette en une seule opération, i.e. facilement et rapidement. En effet, il n'est pas nécessaire de raccorder entre eux les tubes de la tubulure lors de la mise en place ou du remplacement de la cassette comprise dans un dispositif selon l'invention. Lors d'une telle mise en place ou d'un tel remplacement, il est ainsi simplement nécessaire de raccorder les tubes aux autres équipements de manipulation ou d'analyse de fluide pouvant être compris dans un appareil de traitement de fluide comprenant un dispositif de traitement de fluide selon l'invention. De plus, le fait que la tubulure comprise dans la cassette soit préassemblée permet de limiter ou réduire le risque d'erreur lors de la mise en place ou du remplacement d'une telle tubulure dans un dispositif selon l'invention en limitant ou réduisant le nombre d'opérations nécessaires à une telle mise en place ou à un tel remplacement. En outre, le fait d'avoir une tubulure préassemblée dans la cassette permet d'extraire facilement et rapidement la tubulure du dispositif selon l'invention, avec un risque réduit de fuite ou de perte de liquide, ce qui est avantageux, en particulier dans le cas d'un liquide radioactif.

En outre, le dispositif selon l'invention est générique. Ceci signifie que la ou les vannes à pincement comprises dans le dispositif selon l'invention peuvent être couplées avec différentes configurations de tubulure. Autrement dit, la ou les vannes à pincement comprises dans le dispositif selon l'invention peuvent être couplées avec différentes cassettes, chaque cassette comprenant une tubulure préassemblée selon une configuration spécifique. Ceci est rendu possible par le fait que la cassette comprend la tubulure mais ne comprend pas de vanne à pincement. Autrement dit, le fait que la tubulure soit préassemblée dans la cassette permet de découpler la configuration de la tubulure de la configuration des vannes à pincement. Ceci rend le dispositif selon l'invention générique, i.e. capable d'accueillir différentes configurations de tubulures où chaque configuration de tubulure correspond à une cassette différente. Ainsi, un opérateur souhaitant implémenter un schéma de traitement de fluide (ou schéma fluidique) spécifique sur le dispositif selon l'invention pourra sélectionner une cassette comprenant une tubulure préassemblée et configurée de manière à pouvoir réaliser ce schéma fluidique spécifique, et coupler cette cassette avec la ou les vannes à pincement comprises dans le dispositif, permettant ainsi une implémentation rapide et simple de ce schéma fluidique.

Enfin, lors de l'activation d'une vanne à pincement, aucun effort n'est directement appliqué sur la cassette par l'ergot mobile compris dans la vanne à pincement. En effet, la butée contre laquelle l'ergot mobile compris dans une vanne à pincement vient pincer un tube souple pour y contrôler un débit de fluide n'est pas comprise dans la cassette, mais fait partie de ladite vanne à pincement. Dès lors, la cassette ne doit pas être particulièrement rigide et peut donc être réalisée dans un matériau peu robuste, nécessitant ainsi peu de ressources pour être fabriquée. Une cassette comprise dans un dispositif selon l'invention peut donc être fabriquée à moindre coût, et donc mise en place et remplacée à moindre coût. De plus, comme aucun effort n'est directement appliqué sur la cassette lors de l'activation d'une vanne à pincement, la cassette ne doit pas être maintenue fermement en place lorsqu'elle est couplée mécaniquement avec la ou les vannes à pincement comprises dans le dispositif selon l'invention. Ceci permet de faciliter la mise en place et le remplacement de la cassette comprise dans le dispositif selon l'invention en limitant le nombre d'opérations nécessaires à une telle mise en place ou à un tel remplacement.

Avantageusement, la cassette comprise dans le dispositif selon l'invention ne comprend pas de pièce mécanique mobile. Ceci permet de faciliter la stérilisation de la cassette.

Les inventeurs proposent plusieurs modes de réalisation possibles de l'invention comprenant des caractéristiques optionnelles, où certaines d'entre elles peuvent être combinées.

Selon un mode de réalisation, le dispositif de traitement de fluide selon l'invention comprend en outre une base comprenant l'au moins une vanne à pincement, dans lequel la cassette est couplée mécaniquement avec (ou montée sur) la base de sorte que l'ergot mobile de l'au moins une vanne à pincement décrive une trajectoire essentiellement parallèle à la surface essentiellement plane de la cassette lors d'une activation de l'au moins une vanne à pincement pour pincer le tube souple.

La tubulure comprise dans le dispositif de traitement de fluide selon ce mode de réalisation peut également être mise en place et remplacée facilement, rapidement, et à moindre coût. En effet, la cassette comprenant la tubulure est distincte (ou indépendante) de la base comprenant l'au moins une vanne à pincement. La cassette comprenant la tubulure préassemblée peut être couplée mécaniquement avec (ou montée sur) la base comprenant la vanne à pincement. En particulier, la cassette comprenant la tubulure préassemblée peut être simplement posée sur la base comprenant la vanne à pincement. De préférence, la cassette peut être couplée de manière amovible avec la base. Ainsi, la cassette peut être couplée facilement et rapidement avec la base de sorte à coupler mécaniquement la tubulure comprise dans la cassette avec l'au moins une vanne à pincement comprise dans la base. En particulier, la cassette peut être couplée avec la base sans devoir démonter la base ni une partie de la base. Autrement dit, la cassette comprenant la tubulure préassemblée est prête à l'emploi (« plug and play »). De cette manière, la cassette peut être manipulée, i.e. mise en place et remplacée (ou simplement retirée), indépendamment de l'au moins une vanne à pincement comprise dans la base. Autrement dit, la cassette peut être couplée avec ou insérée dans la base indépendamment (du montage et du démontage) de la vanne à pincement. De même, la cassette peut être découplée ou séparée de la base indépendamment (du montage et du démontage) de la vanne à pincement.

De préférence, la cassette est couplée avec la base de manière amovible et indépendamment de l'au moins une vanne à pincement. Ceci permet de faciliter davantage la mise en place et le remplacement de la tubulure en facilitant la mise en place et le remplacement de la cassette comprenant la tubulure préassemblée.

Selon un mode de réalisation, la cassette comprise dans le dispositif selon l'invention comprend en outre un réseau de canaux de support pour accueillir (ou recevoir) la tubulure préassemblée, ledit réseau de canaux de support comprenant de préférence, pour chaque vanne à pincement, une fente de dégagement permettant le montage de l'ergot mobile et de la butée fixe autour du tube souple pouvant être pincé par la vanne à pincement. En effet, la tubulure pouvant être partiellement ou totalement constituée de tubes souples, il est avantageux que la cassette comprenne un réseau de canaux de support afin de recevoir la tubulure. Un tel réseau de canaux de support permet en effet de maintenir la tubulure selon une disposition prédéfinie souhaitée, facilitant ainsi le couplage de la tubulure comprise dans la cassette avec l'au moins une vanne à pincement comprise dans le dispositif selon l'invention. Un tel réseau de canaux de support peut être réalisé sous forme de rainures pratiquées dans l'épaisseur de la cassette et peut comprendre au moins une ramification. Plus précisément, les canaux de support peuvent être réalisés sous forme de rainures s'étendant longitudinalement le long de la tubulure.

De préférence, une fente de dégagement est aménagée dans la cassette au niveau de chaque section de tube souple de la tubulure destinée à être pincée par une vanne à pincement. Cette fente de dégagement permet de recevoir, pour une vanne à pincement, la butée fixe ainsi que l'ergot mobile venant se placer de part et d'autre d'une section de tube souple pouvant être pincée par la vanne, permettant ainsi de faciliter le couplage de la cassette avec l'au moins une vanne à pincement comprise dans le dispositif selon l'invention. Une telle fente de dégagement peut être réalisée sous la forme d'un trou, de préférence un trou ovalisé, pratiqué dans l'épaisseur de la cassette, un tel trou pouvant être par exemple un trou borgne ou un trou traversant l'épaisseur de la cassette.

De préférence, la tubulure préassemblée est maintenue dans le réseau de canaux de support par serrage. En effet, la tubulure pouvant être partiellement ou totalement constituée de tubes souples, il est possible de prévoir une tubulure dont le diamètre extérieur soit ajusté à la largeur des canaux de support de sorte que la tubulure puisse être maintenue dans le réseau de canaux de support par serrage. Cette configuration permet de limiter les ressources nécessaires au maintien de la tubulure dans le réseau de canaux de support. Cette configuration permet également de faciliter la mise en place de la tubulure dans la cassette.

De manière générale, d'autres modes de maintien de la tubulure dans la cassette sont possibles. Ainsi, la tubulure peut être maintenue dans ou sur la cassette au moyen de crochets, au moyen d'encoches ou de rainures dans lesquelles tout ou partie de la tubulure peut être insérée, ou encore par d'autres moyens.

Selon un mode de réalisation, la cassette comprise dans le dispositif selon l'invention est essentiellement constituée d'un polymère thermoplastique. Comme décrit ci-avant, lors de l'activation d'une vanne à pincement, aucun effort n'est directement appliqué sur la cassette par l'ergot mobile compris dans la vanne à pincement. Dès lors, la cassette ne doit pas être particulièrement rigide et peut donc être réalisée dans un matériau peu robuste, nécessitant ainsi peu de ressources pour être fabriquée. Un polymère thermoplastique, comme par exemple le polyméthacrylate de méthyle (PMMA), le polycarbonate (PC), ou encore le polypropylene (PP), constitue un tel matériau peu robuste et présente notamment les avantages suivants. Un polymère thermoplastique peut être peu onéreux et facile à mettre en forme. Une cassette essentiellement constituée d'un polymère thermoplastique peut être ainsi fabriquée en grande série, par exemple par moulage, en nécessitant peu de matière et pour un faible coût unitaire de fabrication. Une telle cassette peut donc être adaptée pour un faible nombre d'utilisations, voire un usage unique, par exemple dans le cas où une telle cassette est amenée à être remplacée fréquemment. De préférence, le polymère thermoplastique dont la cassette comprise dans le dispositif selon l'invention est essentiellement constituée présente l'une quelconque ou plusieurs des caractéristiques suivantes : ledit polymère thermoplastique est translucide, et de préférence transparent, afin de pouvoir notamment voir la tubulure comprise dans la cassette, contrôler l'assemblage de ladite tubulure, et visualiser le parcours du fluide dans ladite tubulure ; ledit polymère thermoplastique est non poreux ; ledit polymère thermoplastique est stérilisable, afin que la cassette puisse être réutilisée, limitant ainsi le volume de déchets générés lors d'un remplacement de la tubulure comprise dans le dispositif selon l'invention.

Selon un mode de réalisation, la tubulure préassemblée comprise dans la cassette comprend au moins un connecteur permettant de coupler la tubulure à au moins un dispositif apte à manipuler le fluide ou mesurer une propriété physico-chimique du fluide. On peut citer les exemples suivants de dispositifs aptes à manipuler un fluide, i.e. permettant de fournir, recevoir, ou déplacer un ou plusieurs fluides dans la tubulure : un flacon d'échantillon de fluide, éventuellement scellé par un septum ; un réservoir de fluide ; une seringue, éventuellement actionnée par un pousse-seringue, et éventuellement équipée d'une aiguille, afin de prélever ou injecter un échantillon de fluide ; une pompe, comme par exemple une pompe péristaltique ; une vanne ; un mélangeur ; un filtre ; un dispositif de chauffe ; un dispositif de refroidissement ; ou d'autres dispositifs. Par ailleurs, on peut citer les exemples suivants de propriétés physico-chimiques du fluide pouvant être mesurées : la température, le pH ; la radioactivité ; la concentration dans le fluide d'un composé chimique ou biochimique, comme par exemple sa concentration en endotoxines ; ou d'autres propriétés. De telles propriétés physico-chimiques peuvent être par exemple mesurées par les dispositifs suivants, auxquels la tubulure peut être couplée : une température peut être mesurée par un thermomètre ; un pH peut être mesuré par un pH-mètre ; une radioactivité peut être mesurée par un scanner de type « radio-TLC » (pour « thin layer chromatography »), ou par un détecteur de type compteur gamma, ou de type compteur multi-canaux, ou encore un compteur à gaz comme une chambre d'ionisation ; une concentration en endotoxines peut être mesurée par un appareil d'analyse par spectrophotométrie. La tubulure peut en outre être couplée à d'autres dispositifs de mesure comme par exemple un appareil de chromatographie, comme un appareil de chromatographie en phase gazeuse, ou encore un appareil de chromatographie en phase liquide, ou d'autres dispositifs. Enfin, on peut citer les exemples suivants de connecteurs permettant de coupler la tubulure à l'un quelconque ou plusieurs des dispositifs susdits, ou à d'autres dispositifs : raccords dits « Luer », tels que « Luer Slip » ou « Luer-Lock », conçus pour permettre un raccordement étanche entre une seringue et une aiguille, et permettant également de raccorder de manière étanche une tubulure à une seringue, ou une tubulure à une aiguille.

Selon un mode de réalisation, la tubulure du dispositif selon l'invention consiste en un réseau de tubes essentiellement cylindriques et d'un diamètre extérieur compris entre 1 mm et 10 mm, de préférence compris entre 2 mm et 3 mm, de préférence égal à 2,4 mm. En effet, le diamètre extérieur de la tubulure est au moins égal à 1 mm afin de permettre un débit de fluide suffisant dans la tubulure, et au plus égal à 10 mm afin de limiter les pertes de fluide, aussi appelées « volumes morts », i.e. le volume de fluide résiduel dans la tubulure après le traitement dudit fluide et qui ne peut pas être facilement extrait de la tubulure et est donc perdu. De préférence, le diamètre extérieur de la tubulure est au moins égal à 2 mm afin de permettre un débit de fluide suffisant dans la tubulure et afin également de faciliter la mise en place de la tubulure dans la cassette. De plus, le diamètre extérieur de la tubulure est au plus égal à 3 mm afin de limiter les pertes de fluide. Enfin, le diamètre extérieur de la tubulure est de préférence égal à 2,4 mm afin de pouvoir s'adapter à des connecteurs disponibles commercialement.

Une vanne à pincement telle que décrite ci-avant, i.e. comprenant un ergot mobile et une butée fixe pour pincer un tube souple pour y contrôler un débit de fluide, peut être qualifiée de vanne à pincement « à deux états », ces deux états étant un état « ouvert », où le tube souple n'est pas pincé et laisse donc passer un débit de fluide maximal, et un état « fermé », où le tube souple est pincé entre l'ergot et la butée et laisse donc passer un débit de fluide minimal, voire où le débit de fluide à travers le tube souple est totalement interrompu, auquel cas ledit débit minimal est nul. Il est important de noter que les deux états susmentionnés ne sont pas les seuls états possibles d'une telle vanne à pincement dite « à deux états », mais qu'ils délimitent plutôt une continuité d'états possibles de ladite vanne, où le tube souple est plus ou moins pincé entre l'ergot et la butée, et correspondants à une continuité de débits possibles entre le débit maximal et le débit minimal susmentionnés. En particulier, l'utilisation d'une vanne à pincement pour contrôler un débit d'un fluide dans un tube souple comme une continuité de débits possibles entre un débit maximal et un débit minimal présente l'avantage de pouvoir contrôler la pression du fluide dans le tube souple en aval de la vanne.

Selon un mode de réalisation, le dispositif de traitement de fluide selon l'invention comprend en outre au moins une vanne à pincement à trois états, une telle vanne à pincement à trois états comprenant un ergot mobile et deux butées fixes disposées de part et d'autre de l'ergot mobile, ladite vanne à pincement à trois états étant configurée pour contrôler un débit d'un fluide dans l'un ou l'autre de deux tubes souples de la tubulure en pinçant l'un des deux tubes souples contre l'une des deux butées fixes par un mouvement de l'ergot mobile suivant une trajectoire essentiellement parallèle à la surface essentiellement plane de la cassette. Les trois états d'une telle vanne à pincement dite « à trois états » et configurée pour contrôler un débit d'un fluide dans l'un ou l'autre d'un premier tube souple et d'un deuxième tube souple sont les suivants. Dans un premier état, aucun des deux tubes n'est pincé et chacun des deux tubes laisse donc passer un débit de fluide maximal. Dans un deuxième état, le premier tube souple est pincé entre l'ergot et la première des deux butées fixes et le débit de fluide traversant ledit premier tube souple est réduit à un débit de fluide minimal, voire même est totalement interrompu, auquel ledit débit minimal est nul, tandis que le deuxième tube souple n'est pas pincé et laisse donc passer un débit de fluide maximal. Dans un troisième état, le deuxième tube souple est pincé entre l'ergot et la deuxième des deux butées fixes et le débit de fluide traversant ledit deuxième tube souple est réduit à un débit de fluide minimal, voire même est totalement interrompu, auquel ledit débit minimal est nul, tandis que le premier tube souple n'est pas pincé et laisse donc passer un débit de fluide maximal.

Par ailleurs, la remarque formulée ci-avant concernant la continuité d'états possibles pour une vanne à pincement dite « à deux états » s'applique également, *mutatis mutandis,* à une vanne à pincement dite « à trois états », i.e. lesdits états ne sont pas les seuls états possibles d'une telle vanne à pincement à trois états mais délimitent plutôt une continuité d'états possibles de ladite vanne, où l'un des deux tubes souples est plus ou moins pincé entre l'ergot et l'une des deux butées, permettant ainsi une continuité de débits possibles entre le débit maximal et le débit minimal susmentionnés. Enfin, on peut noter que comme une telle vanne à pincement à trois états ne comprend qu'un seul ergot mobile, elle n'offre la possibilité de réduire voire d'interrompre un débit d'un fluide que dans un seul de l'un ou l'autre des deux tubes souples à la fois.

L'utilisation d'une vanne à pincement dite « à trois états » offre comme avantage de pouvoir augmenter les configurations possibles d'un schéma fluidique implémenté par un dispositif selon l'invention pour un nombre donné de vannes à pincement et/ou une longueur totale de tubes donnée, ou bien de pouvoir réaliser une configuration donnée d'un schéma fluidique en utilisant un nombre réduit de vannes à pincement et/ou en utilisant une longueur totale de tubes réduite, permettant ainsi de réduire les ressources, les coûts, les points de défaillance possibles, et la maintenance nécessaire pour un tel dispositif.

Selon un mode de réalisation, le dispositif de traitement de fluide selon l'invention comprend en outre au moins une vanne à pincement à plusieurs voies consistant en un assemblage de plusieurs vannes à pincement. Dans le cadre du présent document, un « port », également appelé une « voie », d'une vanne à pincement est défini comme un point d'entrée et/ou de sortie d'un fluide dans une telle vanne à pincement. De manière générale, il est possible qu'un port corresponde à un point d'entrée et de sortie d'un fluide dans un vanne à pincement, auquel cas ce port sera qualifié de port « bidirectionnel ». Une vanne à pincement telle que décrite ci-avant et permettant de contrôler un débit d'un fluide dans un tube souple par pincement dudit tube souple, comporte deux ports et peut ainsi être qualifiée de vanne « à deux voies ». Dans une telle vanne à pincement à deux voies, l'un des deux ports peut être un port d'entrée et l'autre port un port de sortie, ou bien les deux ports peuvent être des ports bidirectionnels. Par ailleurs, il est possible de réaliser une vanne à pincement avec plus de deux ports, par exemple en assemblant plusieurs vannes à pincement à deux voies. Ainsi, une vanne à pincement dite « à trois voies » et comprenant trois ports, par exemple un port d'entrée de fluide et deux ports de sortie de fluide, peut comprendre deux vannes à pincement à deux voies, chacune de ces deux vannes à pincement à deux voies contrôlant un débit d'un fluide entre le port d'entrée et l'un des deux ports de sortie de la vanne à pincement à trois voies. Ainsi, il est possible de réaliser une vanne à pincement à plusieurs voies en assemblant plusieurs vannes à pincement, en particulier en assemblant plusieurs vannes à pincement à deux voies.

L'utilisation d'une vanne à pincement à plusieurs voies offre comme avantage de pouvoir augmenter les configurations possibles d'un schéma fluidique implémenté par un dispositif selon l'invention pour un nombre donné de vannes à pincement et/ou une longueur totale de tubes donnée, ou bien de pouvoir réaliser une configuration donnée d'un schéma fluidique en utilisant un nombre réduit de vannes à pincement et/ou en utilisant une longueur totale de tubes réduite, permettant ainsi de réduire les ressources, les coûts, les points de défaillance possibles, et la maintenance nécessaire pour un tel dispositif.

De préférence, l'au moins une vanne à pincement à plusieurs voies comprise dans le dispositif selon l'invention consiste en une vanne à pincement à quatre voies, formée par l'assemblage de quatre vannes à pincement. En effet, une vanne à pincement à quatre voies formée par l'assemblage de quatre vannes à pincement peut être réalisée de manière compacte, limitant ainsi l'encombrement d'une telle vanne à pincement à quatre voies. De plus, une ou plusieurs d'une telle vanne à pincement à quatre voies peuvent être disposées côte à côte, formant ainsi un ensemble de vannes compact et modulaire, permettant de réaliser un grand nombre de configurations possibles d'un schéma fluidique implémenté par un dispositif selon l'invention.

De préférence, au moins une vanne à pincement comprise dans l'au moins une vanne à pincement à plusieurs voies comprise dans le dispositif selon l'invention est une vanne à pincement à trois états, comprenant un ergot mobile et deux butées fixes. En effet, l'utilisation d'une vanne à pincement à trois états au sein d'une vanne à pincement à plusieurs voies formée par l'assemblage de plusieurs vannes à pincement permet d'augmenter la compacité et la modularité d'une telle vanne à pincement à plusieurs voies, permettant ainsi de réaliser un plus grand nombre de configurations possibles d'un schéma fluidique implémenté par un dispositif selon l'invention.

Selon un mode de réalisation, l'ergot mobile compris dans une vanne à pincement du dispositif selon l'invention est actionné par un servomoteur. Dans le cadre du présent document, on entend par « servomoteur » un moteur asservi en position et/ou en couple, capable de maintenir une opposition à un effort statique, et dont la position et/ou le couple est vérifié en continu et corrigé en fonction de la mesure. L'utilisation d'un servomoteur pour actionner une vanne à pincement présente notamment comme avantage de pouvoir maintenir plusieurs positions stables de la vanne, contrairement aux vannes à pincement de l'art antérieur équipées d'un ressort de rappel, qui ne peuvent typiquement maintenir qu'une seule position stable si elles ne sont pas alimentées en continu, à savoir la position dans laquelle le ressort de rappel est détendu.

Un autre avantage d'utiliser un servomoteur pour actionner une vanne à pincement pour contrôler un débit d'un fluide dans un tube souple est de permettre un contrôle plus précis du débit du fluide dans le tube. En effet, une vanne à pincement selon l'art antérieur est généralement limitée à deux positions limites, ou états : un état « ouvert » de la vanne, où le tube n'est pas pincé et laisse ainsi passer un débit maximal de fluide, et un état « fermé », où le tube est totalement pincé et où le débit dans le tube est ainsi totalement interrompu. Au contraire, lorsqu'une vanne à pincement est actionnée par un servomoteur, celui-ci permet de maintenir une ou plusieurs positions intermédiaires entre lesdites deux positions limites, permettant ainsi un contrôle plus précis du débit du fluide dans le tube.

Selon un mode de réalisation, au moins un tube souple compris dans la tubulure du dispositif selon l'invention est réalisé en silicone. L'utilisation de silicone comme matériau constituant un tube souple de la tubulure présente notamment comme avantages qu'il s'agit d'un matériau peu onéreux et présentant des propriétés intéressantes pour le traitement de fluides dans le cadre d'applications pharmaceutiques et radiopharmaceutiques nécessitant un haut degré de pureté des fluides traités, comme par exemple une faible réactivité avec les fluides circulant dans le tube, une capacité à être utilisé dans une large plage de températures, pouvant aller de -40°C à +200°C et même au-delà, une bonne résistance mécanique.

Selon un deuxième aspect, un objet de la présente invention est de fournir une appareil de synthèse de composés radiopharmaceutiques comprenant une tubulure pouvant être mise en place et remplacée facilement, rapidement, et à moindre coût.

À cet effet, l'invention propose un appareil de synthèse de composés radiopharmaceutiques comprenant un dispositif de traitement de fluide selon l'invention.

Selon un troisième aspect, un objet de la présente invention est de fournir un procédé de traitement de fluide permettant de mettre en place et remplacer facilement, rapidement, et à moindre coût une tubulure comprise dans un dispositif de traitement de fluide selon le premier aspect de l'invention. Le procédé de traitement de fluide selon le troisième aspect de l'invention est spécialement adapté au dispositif de traitement de fluide selon le premier aspect de l'invention.

À cet effet, l'invention propose un procédé de traitement de fluide comprenant :
- fournir une tubulure comprenant au moins un tube souple pouvant être pincé (ou apte à être pincé) ;
- fournir une cassette présentant (ou ayant) une surface essentiellement plane pour recevoir la tubulure ;
- assembler la tubulure avec la cassette ;
- fournir une vanne à pincement comprenant un ergot mobile et une butée fixe, la vanne à pincement étant apte à pincer le tube souple pour y contrôler un débit de fluide (i.e. pour contrôler un débit de fluide dans ledit tube souple) ;
puis :
- coupler mécaniquement la cassette comprenant la tubulure préassemblée avec la vanne à pincement de sorte que l'ergot mobile de la vanne à pincement décrive une trajectoire essentiellement parallèle à la surface essentiellement plane de la cassette lors d'une activation de la vanne à pincement pour pincer le tube souple.
Après l'étape d'assemblage de la cassette avec la tubulure, la cassette comprend la tubulure préassemblée. Dans le procédé selon l'invention, la tubulure est d'abord assemblée (ou préassemblée) avec la cassette avant que la cassette soit couplée mécaniquement avec la vanne à pincement.

De préférence, le procédé selon l'invention comprend en outre :
- faire circuler un fluide dans la tubulure pour traiter ce fluide.

De préférence, le procédé selon l'invention comprend en outre :
- remplacer la cassette comprenant la tubulure préassemblée.
Le remplacement de la cassette permet de remplacer facilement et rapidement la tubulure préassemblée avec la cassette. En particulier, ce remplacement peut être effectué sans devoir démonter la vanne à pincement. Il peut être avantageux de remplacer la cassette comprenant la tubulure préassemblée afin d'implémenter un nouveau schéma de traitement de fluide correspondant à une nouvelle configuration de la tubulure. Ce nouveau schéma de traitement de fluide peut être réalisé au moyen d'une nouvelle tubulure préassemblée avec une nouvelle cassette. La cassette peut également être remplacée afin de limiter ou réduire un risque de contamination d'un fluide circulant dans la tubulure, comme par exemple une contamination de ce fluide par un fluide ayant circulé précédemment dans la tubulure. La cassette peut également être remplacée afin de remplacer une tubulure qui serait usée ou endommagée.

De préférence, le procédé selon l'invention comprend en outre :
- remplacer périodiquement la cassette comprenant la tubulure préassemblée. Il peut être avantageux de remplacer périodiquement la cassette comprenant la tubulure préassemblée afin de limiter ou réduire un risque de contamination d'un fluide circulant dans la tubulure, comme par exemple une contamination de ce fluide par un fluide ayant circulé précédemment dans la tubulure. La cassette peut également être remplacée périodiquement afin de remplacer une tubulure qui serait usée ou endommagée.

Dans le cas où le dispositif selon le premier aspect de l'invention comprend une base comprenant l'au moins une vanne à pincement, un mode de réalisation du procédé selon le troisième aspect de l'invention peut être exprimé comme suit : procédé de traitement de fluide comprenant :
- fournir une tubulure comprenant au moins un tube souple pouvant être pincé (ou apte à être pincé) ;
- fournir une cassette présentant (ou ayant) une surface essentiellement plane pour recevoir la tubulure ;
- assembler la tubulure avec la cassette ;
- fournir une base comprenant une vanne à pincement, la vanne à pincement comprenant un ergot mobile et une butée fixe, la vanne à pincement étant apte à pincer le tube souple pour y contrôler un débit de fluide (i.e. pour contrôler un débit de fluide dans ledit tube souple) ;
puis :
- coupler mécaniquement la cassette comprenant la tubulure préassemblée avec la base de sorte que l'ergot mobile de la vanne à pincement décrive une trajectoire essentiellement parallèle à la surface essentiellement plane de la cassette lors d'une activation de la vanne à pincement pour pincer le tube souple.

L'ensemble des modes de réalisation possibles ainsi que l'ensemble des avantages du dispositif selon le premier aspect de l'invention s'appliquent *mutatis mutandis* aux autres aspects de l'invention.

### Brève description des figures

D'autres caractéristiques et avantages de la présente invention apparaîtront à la lecture de la description détaillée qui suit et pour la compréhension de laquelle on se reportera aux figures annexées parmi lesquelles :
- les Figs. 1a, 1b, 1c, 1d illustrent de manière schématique une vanne à pincement à deux états selon l'invention ;
- les Figs. 2a, 2b, 2c illustrent de manière schématique une vanne à pincement à trois états selon l'invention ;
- la Fig. 3a illustre de manière schématique une vanne à pincement à plusieurs voies selon l'invention et formée par l'assemblage de plusieurs vannes à pincement, tandis que la Fig. 3b illustre de manière schématique un dispositif de traitement de fluide selon l'invention et comprenant une telle vanne à pincement à plusieurs voies ;
- les Figs. 4a, 4b sont une représentation schématique d'une vanne à pincement à plusieurs voies selon l'invention et actionnée par des servomoteurs ;
- la Fig. 5 est une représentation schématique d'un dispositif de traitement de fluide selon l'invention comprenant une cassette comprenant une tubulure préassemblée, et plusieurs vannes à pincement à plusieurs voies ;
- la Fig. 6 est une représentation schématique d'un appareil de synthèse de composés radiopharmaceutiques comprenant un dispositif de traitement de fluide selon l'invention.

Les dessins des figures ne sont pas à l'échelle. Généralement, des éléments semblables sont dénotés par des références semblables dans les figures. Dans le cadre du présent document, des éléments identiques ou analogues peuvent porter les mêmes références. En outre, la présence de numéros de référence aux dessins ne peut être considérée comme limitative, y compris lorsque ces numéros sont indiqués dans les revendications.

### Description détaillée de modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants. En outre, les fonctions décrites peuvent être réalisées par d'autres structures que celles décrites dans le présent document.

L'usage, dans ce document, du verbe « comprendre », de ses variantes, ainsi que ses conjugaisons, ne peut en aucune façon exclure la présence d'éléments autres que ceux mentionnés. L'usage, dans ce document, de l'article indéfini « un », « une », ou de l'article défini « le », « la » ou « l' », pour introduire un élément n'exclut pas la présence d'une pluralité de ces éléments.

Dans le cadre du présent document, les termes « premier », « deuxième », « troisième », etc. servent uniquement à différencier différents éléments et n'impliquent pas d'ordre entre ces éléments.

En outre, les modes de réalisation qualifiés de « préférés » doivent être interprétés comme des exemples de mise en œuvre de l'invention plutôt que comme limitant la portée de l'invention.

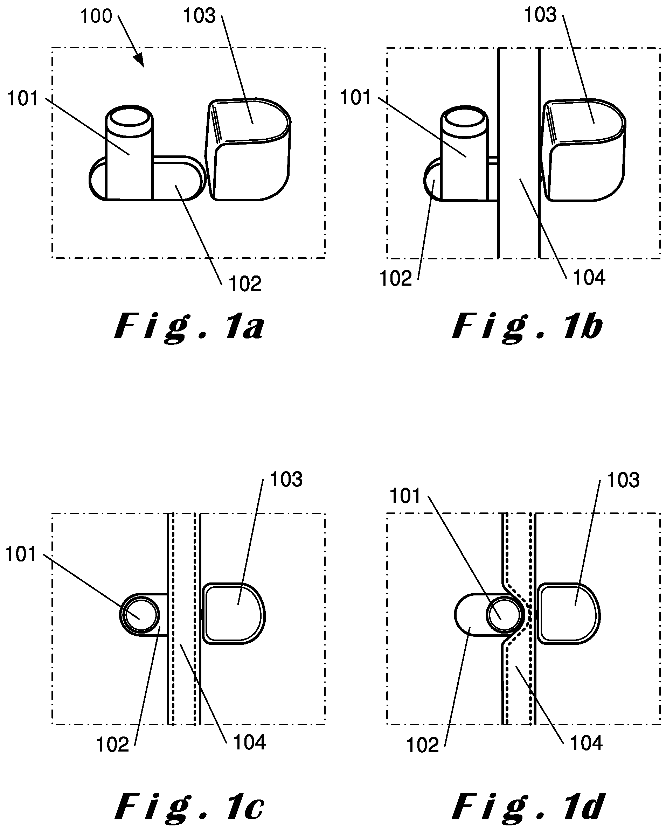

Les Figs. 1a, 1b, 1c, 1d illustrent de manière schématique une vanne à pincement à deux états 100 selon un mode de réalisation possible de l'invention. Comme représenté sur la Fig. 1a, la vanne à pincement à deux états 100 selon l'invention comprend un ergot mobile 101 pouvant se déplacer dans une encoche 102, ainsi qu'une butée fixe 103. Comme représenté sur la Fig. 1b, un tube souple 104 peut venir se placer entre l'ergot 101 et la butée 103 en vue d'être pincé contre la butée 103 par un mouvement de l'ergot 101 dans l'encoche 102 pour contrôler un débit d'un fluide dans le tube 104. La Fig. 1c illustre un état « ouvert » de la vanne à pincement à deux états 100 selon l'invention, où le tube souple 104 n'est pas pincé et laisse donc passer un débit de fluide maximal. La Fig. 1d illustre un état « fermé » de la vanne à pincement à deux états 100 selon l'invention, où le tube souple 104 est pincé entre l'ergot 101 et la butée 103 et où le débit de fluide à travers le tube souple 104 est totalement interrompu.

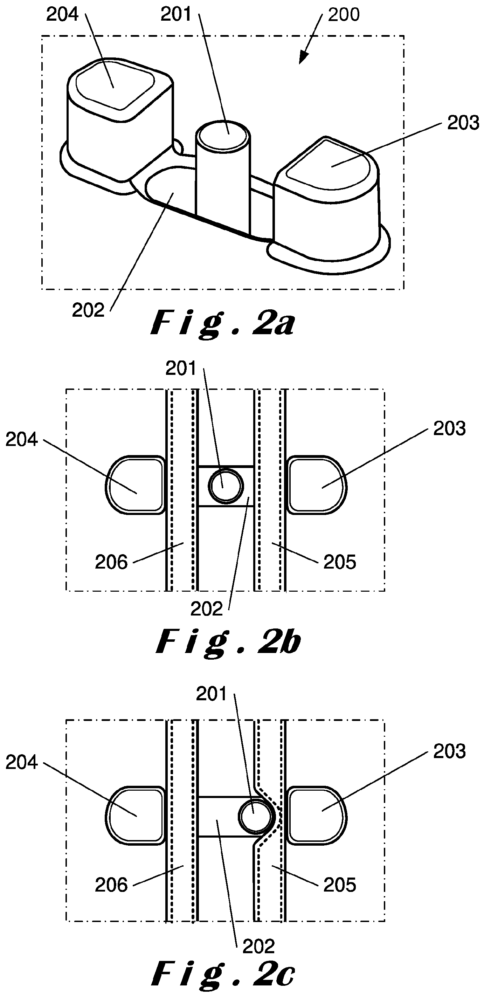

Les Figs. 2a, 2b, 2c illustrent de manière schématique une vanne à pincement à trois états 200 selon un mode de réalisation possible de l'invention. Comme représenté sur la Fig. 2a, la vanne à pincement à trois états 200 selon l'invention comprend un ergot mobile 201 pouvant se déplacer dans une encoche 202, ainsi que deux butées fixes 203, 204 disposées de part et d'autre de l'ergot 201. Comme représenté sur les Figs. 2b et 2c, deux tubes souples 205, 206 peuvent venir se placer entre l'ergot 201 et chacune des deux butées 203, 204 afin que l'ergot 201 puisse pincer l'un des deux tubes souples 205, 206 contre l'une des deux butées 203, 204 pour contrôler un débit de fluide dans ce tube. La Fig. 2b illustre un premier état de la vanne à pincement à trois états 200 selon l'invention, où aucun des deux tubes 205, 206 n'est pincé et où chacun des deux tubes 205, 206 laisse donc passer un débit de fluide maximal. La Fig. 2c illustre un deuxième état de la vanne à pincement à trois états 200 selon l'invention, où le tube 205 est pincé entre l'ergot 201 et la butée 203 et où le débit de fluide à travers le tube 205 est totalement interrompu, tandis que le tube 206 n'est pas pincé et laisse donc passer un débit de fluide maximal.

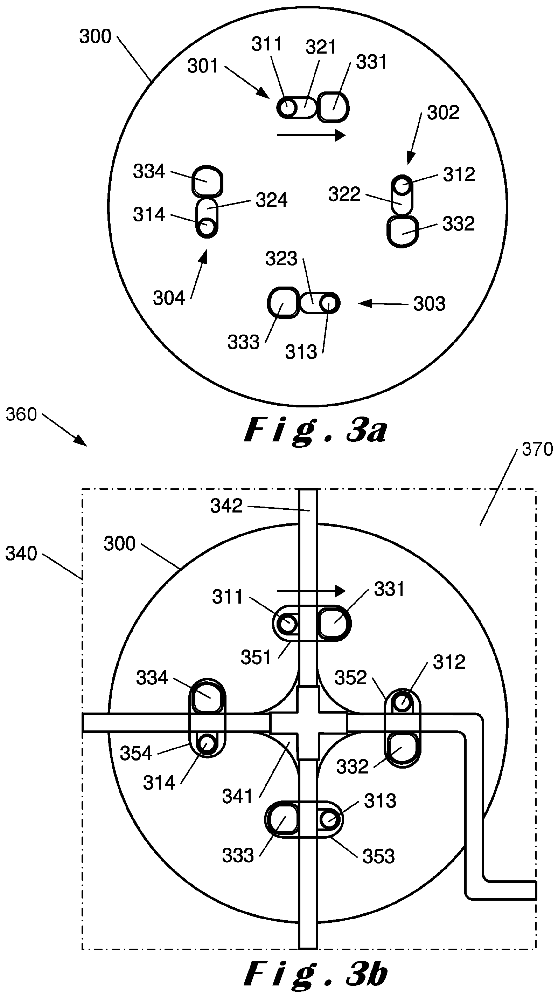

La Fig. 3a illustre de manière schématique une vanne à pincement à plusieurs voies 300 selon un mode de réalisation possible de l'invention et formée par l'assemblage de plusieurs vannes à pincement 301, 302, 303, 304, tandis que la Fig. 3b illustre de manière schématique un dispositif 360 de traitement de fluide selon un mode de réalisation possible de l'invention et comprenant la vanne à pincement à plusieurs voies 300. Plus précisément, la Fig. 3a illustre une vanne à pincement à quatre voies 300 selon l'invention et formée par l'assemblage de quatre vannes à pincement 301, 302, 303, 304 selon l'invention, chacune de ces quatre vannes à pincement 301, 302, 303, 304 comprenant un ergot mobile 311, 312, 313, 314 pouvant se déplacer dans une encoche 321, 322, 323, 324, ainsi qu'une butée fixe 331, 332, 333, 334. La flèche présente à côté de la vanne à pincement 301 illustre la direction le long de laquelle l'ergot 311 peut se déplacer dans l'encoche 321 vers la butée 331. La Fig. 3b illustre un dispositif 360 de traitement de fluide selon l'invention et comprenant la vanne à pincement à quatre voies 300 ainsi qu'une cassette 340 comprenant un réseau de canaux de support 341 comprenant une tubulure 342. La cassette 340 est de préférence essentiellement constituée d'un polymère thermoplastique. De plus, la cassette 340 est de préférence transparente. La cassette 340 présente une surface 370 essentiellement plane pour recevoir la tubulure 342. Le réseau de canaux de support 341 comprend en outre des fentes de dégagement 351, 352, 353, 354 afin de recevoir l'ergot mobile 311, 312, 313, 314 et la butée fixe 331, 332, 333, 334 compris dans chacune des vannes à pincement 301, 302, 303, 304. Chacune des fentes de dégagement 351, 352, 353, 354 est réalisée sous la forme d'un trou ovalisé pratiqué dans l'épaisseur de la cassette 310. Ces fentes de dégagement 351, 352, 353, 354 permettent de faciliter le couplage entre la tubulure 342 comprise dans la cassette 340 et les vannes à pincement 301, 302, 303, 304. Enfin, comme sur la Fig. 3a, une flèche illustre la direction le long de laquelle l'ergot 311 peut se déplacer vers la butée 331 pour pincer un tube souple de la tubulure 342 en décrivant une trajectoire essentiellement parallèle à la surface 370 essentiellement plane de la cassette 340.

La vanne à pincement à plusieurs voies 300 est un exemple d'une base 300 pouvant être couplée mécaniquement avec une cassette 340 comprenant une tubulure 342 préassemblée.

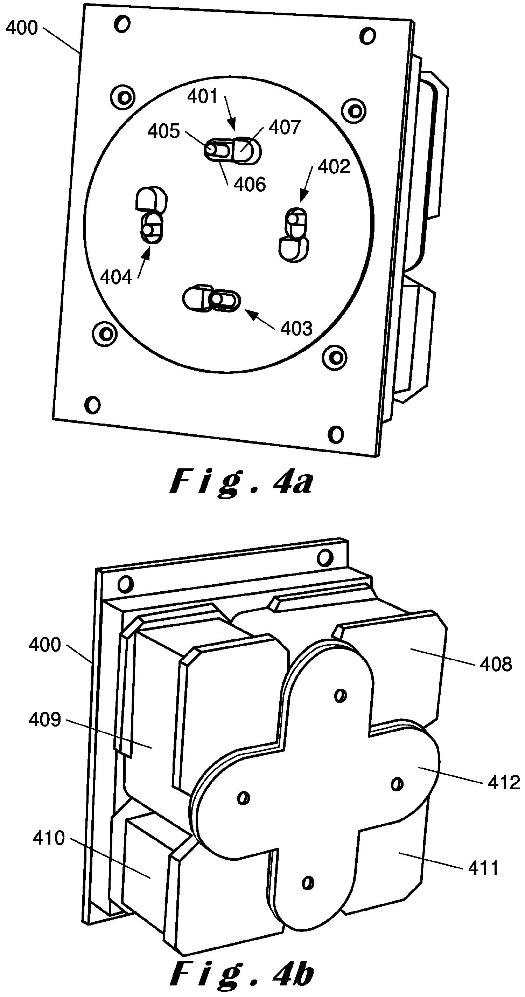

Les Figs. 4a et 4b sont une représentation schématique d'une vanne à pincement à plusieurs voies 400 selon un mode de réalisation possible de l'invention et actionnée par des servomoteurs. Plus précisément, les Figs. 4a et 4b sont une vue en perspective de la vanne à pincement à quatre voies 300 représentée à la Fig. 3a, dans un cas où cette vanne à pincement 300 est actionnée par des servomoteurs. La vanne à pincement 400 est donc également un exemple d'une base pouvant être couplée mécaniquement avec une cassette 340 comprenant une tubulure 342 préassemblée.

La Fig. 4a présente une vue avant de la vanne à pincement à quatre voies 400, tandis que la Fig. 4b présente une vue arrière de la vanne à pincement à quatre voies 400. La vanne à pincement 400 comprend quatre vannes à pincement 401, 402, 403, 404, correspondant respectivement aux vannes à pincement 301, 302, 303, 304 représentées sur les Figs. 3a et 3b. Chacune des vannes à pincement 401, 402, 403, 404 comprend un ergot mobile pouvant se déplacer dans une encoche, ainsi qu'une butée fixe, comme par exemple l'ergot mobile 405, l'encoche 406, et la butée fixe 407 pour la vanne à pincement 401. Les vannes à pincement 401, 402, 403, 404 sont respectivement actionnées par les servomoteurs 408, 409, 410, 411, ces servomoteurs étant maintenus par un support arrière 412.

Une vanne à pincement à plusieurs voies 400 telle qu'illustrée sur les Figs. 4a et 4b présente plusieurs avantages. Tout d'abord, une telle vanne à pincement 400 présente un encombrement réduit. En effet, les vannes à pincement 401, 402, 403, 404 comprises dans la vanne à pincement 400 peuvent être placées relativement proches les unes des autres, permettant ainsi de réaliser de manière compacte une vanne à pincement à plusieurs voies.

De plus, les vannes à pincement 401, 402, 403, 404 sont situées sur un même plan, ce qui permet de coupler en une seule opération la tubulure comprise dans une cassette avec l'ensemble desdites vannes à pincement, comme décrit ci-avant. La vanne à pincement à plusieurs voies 400 est un exemple d'une base comprenant une pluralité de vannes à pincement 401, 402, 403, 404 et avec laquelle une cassette 340 comprenant une tubulure 342 préassemblée et présentant une surface 370 essentiellement plane pour recevoir la tubulure 342 peut être couplée mécaniquement. Plus précisément, la cassette 340 peut être couplée avec cette base de sorte que l'ergot mobile de chacune des vannes à pincement 401, 402, 403, 404 comprises dans la base décrive une trajectoire essentiellement parallèle à la surface 370 essentiellement plane de la cassette 340 lors d'une activation des vannes à pincement 401, 402, 403, 404 pour pincer un tube souple de la tubulure 342. De préférence, la cassette 340 est couplée de manière amovible avec cette base.

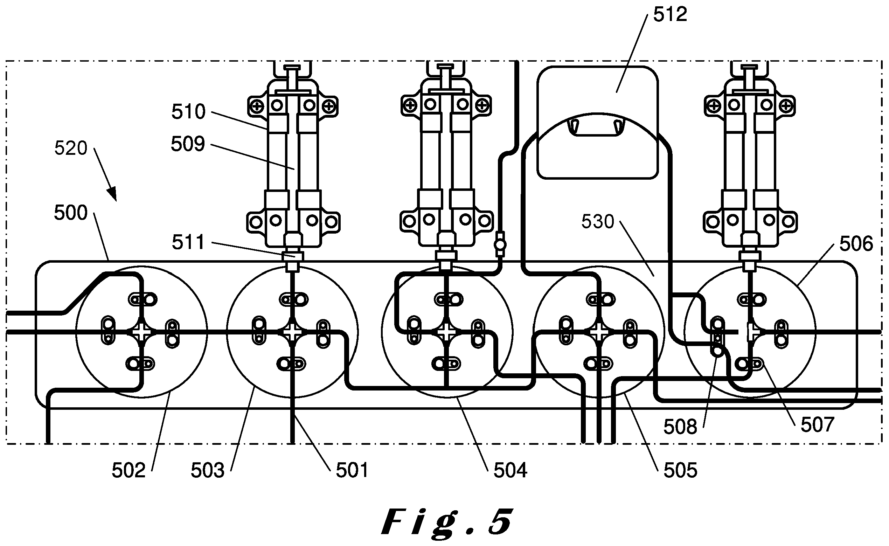

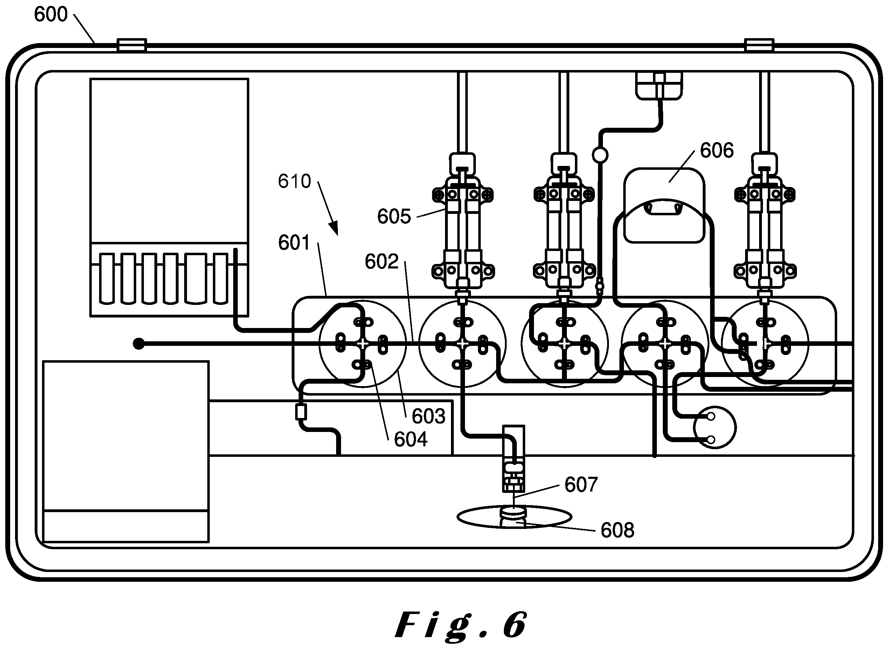

Enfin, les actionneurs (servomoteurs) 408, 409, 410, 411 étant disposés de manière compacte à l'arrière de la vanne à pincement 400, il est possible d'agencer côte à côte plusieurs exemplaires de cette vanne à pincement 400 pour former un ensemble de vannes compact et modulaire, comme illustré à la Fig. 5 et à la Fig. 6.

La Fig. 5 est une représentation schématique d'un dispositif 520 de traitement de fluide selon un mode de réalisation possible de l'invention et comprenant une cassette 500 comprenant une tubulure 501 préassemblée et couplée mécaniquement à plusieurs vannes à pincement à plusieurs voies 502, 503, 504, 505, 506. La cassette 500 présente une surface 530 essentiellement plane pour recevoir la tubulure 501. Les vannes à pincement à plusieurs voies 502, 503, 504, 505, 506 forment une base avec laquelle la cassette 500 comprenant la tubulure 501 préassemblée peut être couplée mécaniquement. Chacune des vannes à pincement à plusieurs voies 502, 503, 504, 505, 506 peut comprendre une ou plusieurs vannes à pincement à deux états 507 et/ou une ou plusieurs vannes à pincement à trois états 508. De plus, la tubulure 501 comprise dans la cassette 500 peut être couplée à un ou plusieurs dispositifs aptes à manipuler un fluide ou mesurer une propriété physico-chimique du fluide, comme par exemple : une seringue 509 pouvant être actionnée par un pousse-seringue 510 et couplée à la tubulure 501 au moyen d'un connecteur tel qu'un raccord dit « Luer » 511 ; ou encore une pompe péristaltique 512.

La Fig. 6 est une représentation schématique d'un exemple d'un appareil 600 de synthèse de composés radiopharmaceutiques comprenant un dispositif 610 de traitement de fluide selon l'invention. Le dispositif 610 de traitement de fluide de la Fig. 6 correspond au dispositif 520 de traitement de fluide de la Fig. 5. Le dispositif 610 comprend une cassette 601 comprenant une tubulure 602 couplée mécaniquement à une pluralité de vannes à pincement à plusieurs voies 603, chacune de ces vannes à pincement à plusieurs voies 603 pouvant comprendre une ou plusieurs vannes à pincement 604. La pluralité de vannes à pincement à plusieurs voies 603 est un exemple d'une base avec laquelle la cassette 601 comprenant la tubulure 602 préassemblée peut être couplée mécaniquement. De plus, la tubulure 602 comprise dans la cassette 601 peut être couplée à un ou plusieurs dispositifs aptes à manipuler un fluide ou mesurer une propriété physico-chimique du fluide, comme par exemple : une seringue pouvant être actionnée par un pousse-seringue 605 ; une pompe péristaltique 606 ; une aiguille 607 permettant d'injecter ou de prélever un échantillon de fluide dans un flacon 608.

En résumé, l'invention concerne un dispositif 360, 520 de traitement de fluide comprenant : une cassette 340, 500 comprenant une tubulure 342, 501 préassemblée, la cassette 340, 500 présentant une surface 370, 530 essentiellement plane pour recevoir la tubulure 342, 501, la tubulure 342, 501 comprenant au moins un tube souple 104 pouvant être pincé ; au moins une vanne à pincement 100, 200 comprenant un ergot mobile 101 et une butée fixe 103 pour pincer le tube souple 104 pour y contrôler un débit de fluide ; dans lequel l'au moins une vanne à pincement 100, 200 est couplée mécaniquement avec la cassette 340, 500 de sorte que l'ergot mobile 101 de l'au moins une vanne à pincement 100, 200 décrive une trajectoire essentiellement parallèle à la surface 370, 530 essentiellement plane de la cassette lors d'une activation de l'au moins une vanne à pincement 100, 200 pour pincer le tube souple 104. La tubulure 342, 501 comprise dans le dispositif 360, 520 de traitement de fluide selon l'invention peut être mise en place et remplacée facilement, rapidement, et à moindre coût. L'invention concerne également un procédé de traitement de fluide permettant de mettre en place et remplacer facilement, rapidement, et à moindre coût une tubulure 342, 501 comprise dans un dispositif 360, 520 de traitement de fluide selon l'invention.

La présente invention a été décrite en relation avec des modes de réalisation spécifiques, qui ont une valeur purement illustrative et ne doivent pas être considérés comme limitatifs. D'une manière générale, il apparaîtra de façon évidente à l'homme du métier que la présente invention n'est pas limitée aux réalisations et aux exemples illustrés et/ou décrits ci-dessus, mais que sa portée est plus largement définie par les revendications ci-après introduites.

## Revendications

1. Dispositif (360, 520) de traitement de fluide comprenant :
- une cassette (340, 500) comprenant une tubulure (342, 501) préassemblée, la cassette (340, 500) présentant une surface (370, 530) essentiellement plane pour recevoir la tubulure (342, 501), la tubulure (342, 501) comprenant au moins un tube souple (104) pouvant être pincé ;
- au moins une vanne à pincement (100, 200) comprenant un ergot mobile (101) et une butée fixe (103) pour pincer le tube souple (104) pour y contrôler un débit de fluide ;
dans lequel l'au moins une vanne à pincement (100, 200) est couplée mécaniquement avec la cassette (340, 500) de sorte que l'ergot mobile (101) de l'au moins une vanne à pincement (100, 200) décrive une trajectoire essentiellement parallèle à la surface (370, 530) essentiellement plane de la cassette lors d'une activation de l'au moins une vanne à pincement (100, 200) pour pincer le tube souple (104).

2. Dispositif (360, 520) de traitement de fluide selon la revendication précédente et comprenant en outre une base (300) comprenant l'au moins une vanne à pincement (100, 200), dans lequel la cassette (340, 500) est couplée mécaniquement avec la base (300) de sorte que l'ergot mobile (101) de l'au moins une vanne à pincement (100, 200) décrive une trajectoire essentiellement parallèle à la surface (370, 530) essentiellement plane de la cassette lors d'une activation de l'au moins une vanne à pincement (100, 200) pour pincer le tube souple (104).

3. Dispositif (360, 520) de traitement de fluide selon la revendication précédente, dans lequel la cassette (340, 500) est couplée avec la base (300) de manière amovible et indépendamment de l'au moins une vanne à pincement (100, 200).

4. Dispositif (360, 520) de traitement de fluide selon l'une quelconque des revendications précédentes, dans lequel la cassette (340) comprend en outre un réseau de canaux de support (341) pour accueillir la tubulure (342) préassemblée, ledit réseau de canaux de support (341) comprenant de préférence, pour chaque vanne à pincement (301, 302, 303, 304), une fente de dégagement (351, 352, 353, 354) permettant le montage de l'ergot mobile (311, 312, 313, 314) et de la butée fixe (331, 332, 333, 334) autour du tube souple (104).

5. Dispositif (360, 520) de traitement de fluide selon l'une quelconque des revendications précédentes, dans lequel la cassette (340, 500) est essentiellement constituée d'un polymère thermoplastique.

6. Dispositif (360, 520) de traitement de fluide selon l'une quelconque des revendications précédentes, dans lequel la tubulure (501) préassemblée comprise dans la cassette (500) comprend au moins un connecteur (511) permettant de coupler la tubulure (501) à au moins un dispositif (509, 512) apte à manipuler le fluide ou mesurer une propriété physico-chimique du fluide.

7. Dispositif (360, 520) de traitement de fluide selon l'une quelconque des revendications précédentes, dans lequel la tubulure (342, 501) consiste en un réseau de tubes essentiellement cylindriques et d'un diamètre extérieur compris entre 1 mm et 10 mm, de préférence compris entre 2 mm et 3 mm, de préférence égal à 2,4 mm.

8. Dispositif (360, 520) de traitement de fluide selon l'une quelconque des revendications précédentes et comprenant en outre au moins une vanne à pincement à trois états (200, 508), une telle vanne à pincement à trois états (200, 508) comprenant un ergot mobile (201) et deux butées fixes (203, 204) disposées de part et d'autre de l'ergot mobile (201), ladite vanne à pincement à trois états (200, 508) étant configurée pour contrôler un débit d'un fluide dans l'un ou l'autre de deux tubes souples (205, 206) de la tubulure (501) en pinçant l'un des deux tubes souples (205, 206) contre l'une des deux butées fixes (203, 204) par un mouvement de l'ergot mobile (201) suivant une trajectoire essentiellement parallèle à la surface (370, 530) essentiellement plane de la cassette (340, 500).

9. Dispositif (360, 520) de traitement de fluide selon l'une quelconque des revendications précédentes et comprenant en outre au moins une vanne à pincement à plusieurs voies (300) consistant en un assemblage de plusieurs vannes à pincement (301, 302, 303, 304).

10. Dispositif (360, 520) de traitement de fluide selon la revendication précédente, dans lequel l'au moins une vanne à pincement à plusieurs voies (300) consiste en une vanne à pincement à quatre voies, formée par l'assemblage de quatre vannes à pincement (301, 302, 303, 304).

11. Dispositif (360, 520) de traitement de fluide selon l'une quelconque des deux revendications précédentes, dans lequel au moins une vanne à pincement comprise dans l'au moins une vanne à pincement à plusieurs voies (300) est une vanne à pincement à trois états (508), comprenant un ergot mobile et deux butées fixes.

12. Dispositif (360, 520) de traitement de fluide selon l'une quelconque des revendications précédentes, dans lequel l'ergot mobile (405) compris dans une vanne à pincement (401) est actionné par un servomoteur (408).

13. Appareil (600) de synthèse de composés radiopharmaceutiques comprenant un dispositif (610) de traitement de fluide selon l'une quelconque des revendications précédentes.

14. Procédé de traitement de fluide comprenant :
- fournir une tubulure (342, 501) comprenant au moins un tube souple (104) pouvant être pincé ;
- fournir une cassette (340, 500) présentant une surface (370, 530) essentiellement plane pour recevoir la tubulure (342, 501) ;
- assembler la tubulure (342, 501) avec la cassette (340, 500) ;
- fournir une vanne à pincement (100, 200) comprenant un ergot mobile (101) et une butée fixe (103), la vanne à pincement (100, 200) étant apte à pincer le tube souple (104) pour y contrôler un débit de fluide ;
puis :
- coupler mécaniquement la cassette (340, 500) avec la vanne à pincement (100, 200) de sorte que l'ergot mobile (101) de la vanne à pincement (100, 200) décrive une trajectoire essentiellement parallèle à la surface (370, 530) essentiellement plane de la cassette lors d'une activation de la vanne à pincement (100, 200) pour pincer le tube souple (104).

15. Procédé selon la revendication précédente et comprenant en outre :
- remplacer la cassette (340, 500) comprenant la tubulure (342, 501) préassemblée.
